# EUROPEAN PATENT SPECIFICATION

(11) **EP 1 942 184 B1**
(45) Date of publication and mention of the grant of the patent: **04.06.2014**
(21) Application number: 06822384.1
(22) Date of filing: 26.10.2006
(51) Int. Cl.: C12N 15/74, C12N 15/52, C12P 7/62, C12N 1/21

(54) **Transformant capable of carrying plasmid stably**
Transformante die ein Plasmid stabil zurückhält
Transformant assurant la stabilité d'un plasmide

(30) Priority: 27.10.2005 JP 2005312921
(43) Date of publication of application: 09.07.2008
(73) Proprietor: KANEKA CORPORATION, Osaka (JP)
(72) Inventor: SATO, Shunsuke, Takasago-shi, Hyogo 676-8688 (JP)
(74) Representative: HOFFMANN EITLE
(86) International application number: PCT/JP2006/321415
(87) International publication number: WO 2007/049716

(56) References cited:
- WO-A1-2004/074476
- WO-A1-2005/098001
- JP-B2- 3 062 459
- US-A- 4 760 022
- US-A- 5 670 343
- US-A- 6 143 518
- TAGHAVI S ET AL: "Identification of a partition and replication region in the Alcaligenes eutrophus megaplasmid pMOL28" MOLECULAR AND GENERAL GENETICS, SPRINGER VERLAG, BERLIN, DE, vol. 250, no. 2, 5 February 1996 (1996-02-05), pages 169-179, XP003011952 ISSN: 0026-8925
- JACKSON J K ET AL: "Effects of recombinant modulation of the phbCAB operon copy number on PHB synthesis rates in Ralstonia eutropha" JOURNAL OF BIOTECHNOLOGY, ELSEVIER SCIENCE PUBLISHERS, AMSTERDAM, NL, vol. 68, no. 1, 5 February 1999 (1999-02-05), pages 49-60, XP004157318 ISSN: 0168-1656
- HAIGERMOSER C ET AL: "Stability of r-microbes: Stabilization of plasmid vectors by the partitioning function of broad-host-range plasmid RP4" JOURNAL OF BIOTECHNOLOGY, ELSEVIER SCIENCE PUBLISHERS, AMSTERDAM, NL, vol. 28, no. 2-3, 1 April 1993 (1993-04-01), pages 291-299, XP023705161 ISSN: 0168-1656 [retrieved on 1993-04-01]
- LEE S Y ET AL: "Construction of plasmids, estimation of plasmid stability, and use of stable plasmids for the production of poly(3-hydroxybutyric acid) by recombinant Escherichia coli" JOURNAL OF BIOTECHNOLOGY, ELSEVIER SCIENCE PUBLISHERS, AMSTERDAM, NL, vol. 32, no. 2, 14 February 1994 (1994-02-14), pages 203-211, XP023794576 ISSN: 0168-1656 [retrieved on 1994-02-14]
- YU HUIMIN ET AL: "Effect of poly(beta-hydroxybutyrate) accumulation on the stability of a recombinant plasmid in Escherichia coli." JOURNAL OF BIOSCIENCE AND BIOENGINEERING, vol. 96, no. 2, August 2003 (2003-08), pages 179-183, XP002524267 ISSN: 1389-1723
- TAGHAVI S. ET AL: 'Identification of a partition and replication region in the Alcaligenes eutrophus megaplasmid pMOL28' MOL. GEN. GENET vol. 250, 1996, pages 169 - 179, XP003011952
- VANDAMME P. ET AL: 'Taxonomy of the genus Cupriavidus: a tale of lost and found' INT. J. SYST. EVOL. MICROBIOL. vol. 54, 2004, pages 2285 - 2289, XP003011953
- VANEECHOUTTE M. ET AL: 'Wautersia gen. nov., a novel genus accommodating the phylogenetic lineage including Ralstonia eutropha and related species, and proposal of Ralstonia [Pseudomonas] syzygii (Roberts et al. 1990) comb. nov.' INT. J. SYST. EVOL. MICROBIOL. vol. 54, 2004, pages 317 - 327, XP003011954
- GORIS J. ET AL: 'Classification of metal-resistant bacteria from industrial biotopes as Ralstonia campinensis sp. nov., Ralstonia meallidurans sp. nov. and Ralstonia basilensis Steinle et al. 1998 emend.' INT. J. SYST. EVOL. MICROBIOL. vol. 51, 2001, pages 1773 - 1782, XP003011955
- MERGEAY M. ET AL: 'Ralstonia metallidurans, a bacterium specifically adapted to toxic metals: towards a catalogue of metal-responsive genes' FEMS MICROBIOLOGY REVIEWS vol. 27, 2003, pages 385 - 410, XP003011956
- MATSUMOTO K. ET AL: 'Enhancement of poly (3-hydroxybutyrate-co-3-hydroxyvalerate) production in the transgenic Arabidopsis thaliana by the in vitro evolved highly active mutants of polyhydroxyalkanoate (PHA) synthase from Aeromonas caviae' BIOMACROMOLECULES vol. 6, July 2005 - August 2005, pages 2126 - 2130, XP003011957
- TSUGE T. ET AL: 'Biosynthesis and compositional regulation of poly[(3-hydroxybutyrate)-co-(3-hydroxyhexan oate)] in recombinant ralstonia eutropha expressing mutated polyhydroxyalkanoate synthase genes' MACROMOLECULAR BIOSCIENCE vol. 4, 2004, pages 238 - 242, XP003011958
- Simon R and Frommer W: "Safety Aspects in Biotechnology" In: Pühler A: "Biotechnology: Genetic Fundamentals and Genetic Engineering", 1993, VCH ISBN: 3527283129 vol. 2, pages 835-853,
- TSUGE TAKEHARU ET AL: "Mutation effects of a conserved alanine (Ala510) in type I polyhydroxyalkanoate synthase from Ralstonia eutropha on polyester biosynthesis", MACROMOLECULAR BIOSCIENCE, WILEY VCH VERLAG, WEINHEIM, DE, vol. 4, no. 10, 20 October 2004 (2004-10-20), pages 963-970, ISSN: 1616-5187, DOI: 10.1002/MABI.200400075 [retrieved on 2004-10-26]
- SIMON SILVER ET AL: "Introduction to a special Festschrift issue celebrating the microbiology of Cupriavidus metallidurans strain CH34", ANTONIE VAN LEEUWENHOEK, KLUWER ACADEMIC PUBLISHERS, DO, vol. 96, no. 2, 24 June 2009 (2009-06-24), pages 113-114, ISSN: 1572-9699, DOI: DOI:10.1007/S10482-009-9357-0
- JACKSON J K ET AL: "Effects of recombinant modulation of the phbCAB operon copy number on PHB synthesis rates in Ralstonia eutropha", JOURNAL OF BIOTECHNOLOGY, ELSEVIER SCIENCE PUBLISHERS, AMSTERDAM, NL, vol. 68, no. 1, 5 February 1999 (1999-02-05), pages 49-60, ISSN: 0168-1656, DOI: DOI:10.1016/S0168-1656(98)00186-2
- SCHWARTZ E ET AL: "A physical map of the megaplasmid pHG1, one of three genomic replicons in Ralstonia eutropha H16", FEMS MICROBIOLOGY LETTERS, WILEY-BLACKWELL PUBLISHING LTD, GB, vol. 201, no. 2, 24 July 2001 (2001-07-24) , pages 213-219, ISSN: 0378-1097 [retrieved on 2001-07-24]
- ROB VAN HOUDT ET AL: "New mobile genetic elements in Cupriavidus metallidurans CH34, their possible roles and occurrence in other bacteria", ANTONIE VAN LEEUWENHOEK, KLUWER ACADEMIC PUBLISHERS, DO, vol. 96, no. 2, 24 April 2009 (2009-04-24) , pages 205-226, ISSN: 1572-9699, DOI: 10.1007/S10482-009-9345-4

### SEQUENCE LISTING

<110> KANEKA corporation
<120> NOVEL PLASMID VECTOR AND TRANSFORMANT CAPABLE OF CARRYING PLASMID STABLY
<130> B050561WO01
<150> JP2005-312921
   <151> 2005-10-27
<160> 19
<170> PatentIn version 3.1
<210> 1
   <211> 20
   <212> DNA
   <213> Artificial
<220>
   <223> Primer
<400> 1
   ctttcagcgc aatgtacacc 20
<210> 2
   <211> 20
   <212> DNA
   <213> Artificial
<220>
   <223> Primer
<400> 2
   ctgtacacgc ctcaaaagga 20
<210> 3
   <211> 20
   <212> DNA
   <213> Artificial
<220>
   <223> Primer
<400> 3
   tttcgttcca ctagacgtca 20
<210> 4
   <211> 20
   <212> DNA
   <213> Artificial
<220>
   <223> Primer
<400> 4
   cgcatcagga aattgtaagc 20
<210> 5
   <211> 20
   <212> DNA
   <213> Artificial
<220>
   <223> Primer
<400> 5
   ccagtgtgct gcaattggcc 20
<210> 6
   <211> 20
   <212> DNA
   <213> Artificial
<220>
   <223> Primer
<400> 6
   ccgttactag tggatccgag 20
<210> 7
   <211> 1223
   <212> DNA
   <213> Cupriavidus metallidurans
<400> 7
   tgtacaccct aggccatggc atgagcattg ttcgtcgtgt ttgccgcgtt ggctgacctg 60
   cggtcgcgca gcatcgctcg caacgccgat aacgtcgaag ccgcctcctt tccccgtttc 120
   tcgagccggc cgcttgctct ctggggagca gcgtagacct cagctttctg gtacgcacca 180
   gcgtgtagtc gccttgcaaa ttcggcgagg gaagttccta ccaagcggga agtgaccgca 240
   ctttggacct cggtgacaag cactgagctt ccgtcgcttt cgacttcgaa atggttgcct 300
   ttttcgtcca cgaaacactg ccgcgctgcc cctgcgagta tcgaacgcac gaaatcgcgg 360
   tcctgcactt ggattcgggc tttgtcttct ttgagtgcct ttgctttgca gacagcactg 420
   aaatcggtgc gggcggtgag tagggcaagc agatacgctt ttggcacgcg tgctttcgca 480
   agactctccc atgtcgcgct tacgacatct gagagaaagt ggccttgctc tttagccttt 540
   cgcatcaacc aaaaaattaa atttacatca agacccaggc cgcgcagacg tgtcaggtcc 600
   tgggggagcc gatcctgctg tcttttttga aaggagtatg ggacacgatc ttctgtaaac 660
   aggactctcg ttttgagaga cggttgcgcc atagaagagg gtggtggaac ggccggctcg 720
   ccgagctgcg ctgtgtcgtc ctcgcccgta cccgtggttt cttcttcttt tcgcgagctt 780
   aagccgagca tctccgtcgc tttttccgtc aggtaaatgt aggctcctgc gaatcggcca 840
   aatcgcttcc gaacttgaac gtcgacggta attaggccca agtcgagcag ttctcgttga 900
   gcacggtacc aagtagccgg cgacattccg gtttcggcac aaagcgtttc ccggtgctta 960
   aagatcttac cggtggggtc atcgttattc gccgtgcggg cgagggcggc aagagctgat 1020
   cgggcgctac gcccgatccc gacgattcga gttgcgcgct cgactgcttc gatagtcttg 1080
   cgtttgaggc gcttgggcgc cggagcaatc agagcgtctg tctggttaag gtgtttttgt 1140
   gggcgagcaa catcctcgcc agcaacggag cgttgcgcga aagtcatatg accttcccat 1200
   tcgtaaaaaa tggttgaagt cgc 1223
<210> 8
   <211> 2848
   <212> DNA
   <213> Cupriavidus metallidurans
<400> 8
   ttgactcagg ttcgcgttcc gctacactcc gactaacgaa tccatgtcga gtggcttcgg    60
   tgtgtagcgg ccgggaaact ggtcagcttg aagttctcaa aagcccacgg ttgccgccgt   120
   gggcttttgt ttttgtagga ttacagtcta gacatggctt aaaacctcga ttcgtagtac   180
   gaagtcctta aaaaacaagc acttagcttt ttgcgtcttc cgacgcagct ttagctaacc   240
   cttccagatg ttcgcgaatg gccgattggg ccgcttccgc ttcttcctcg ctgcggaact   300
   caatgcgcat gactttcttt gcgatacgaa catcgcacca agtcgccttt cccgccttga   360
   ttttgaagcc ggtagatgcg gcaggcctgg tcttaacctg ctcggcctta gtcatccgta   420
   cggcctgctg ttgatcgatc ttcttctcgg ccaacaactt aaccgcttcg acgacccgat   480
   ccccgcgacc gtctttggtc gcccttgcca actcggcgcc agcattcgac ccgagcaggt   540
   ctgggcggct atcgagaagg cttaggacct cgggaggcaa gttccggaac gacaaaatct   600
   ctgagagagt cgagacaggt acaccagcct gttcagctat tgcggcttga gtcttgtctg   660
   ggctgcgaag cagcagttcg tcgaacttcc gaaacttctc gaaatccgtt aaatctgact   720
   gcataaggtt cgcgtagaac gcgcccgtgt cagcctcgtc tgaactaagt tcgccgagca   780
   cgcagcgtat gtgatcgcgc ccaagctcgc ggtacgcgtc tgtccggtga tgcccggaga   840
   caatctcgaa gcctcccgca ggccgagggc atacaatcac aggatgaacg agcttgttgt   900
   gcctgaggtt ttccctcaat tcgacatact tctcgggagg catgaagcgt ctgcggcctg   960
   ggacctcatg cagttggtcg ataggcacat ccactgcgcc gccgctgacg cgggccgact  1020
   caagttctgc tcttagttgc gcgatctcgt tagcctgtcg ctcaaccttc ccttgcatat  1080
   gcattaactg accgggcgca gttcgcggcg cttgcggacc gcgcgcagcg gctttctcca  1140
   agtcttccgc cgtgaccttg atgttcccag cctttgcggc aagcttttct ctcaaactca  1200
   ttcttcaatt ccttgccaaa gcgcgctgac cttgtcgtcc acatagtcga caaactcatc  1260
   gcagggttgg cgaatgcgtc gcatcgtctc ggtgttcgcc tcgctagagg agaggtcata  1320
   gaccgtgcga aattgaatgc tgctgttacg ggcgaggtcc gtctcaggga tctcaatcgg  1380
   cagcacgcgc gacccataga cgccgcgaat ccagtctgcc accaggcgag gagcgttctt  1440
   tttctccatg cgtgtcatga gaacatcgag aaagtcgaac tcctttttag atccctcgct  1500
   ctgctcttcc atgcctgtta ctaggtccga gaagagttgc cagaactgga ccatagacgc  1560
   gaaagccaag gtgtccggga ccaccggtac gatgacgcca tcggcagcga aaatcgcgtt  1620
   gatggtcagg tagctgaggg taggagccgt gtcgaggatg atgtagtcat attcgtcttt  1680
   caacgaatcg aggccgttac ttaacacgcg ctcgaacgga atatggcctt cctctgccgt  1740
   cgcccgagcc ggaagcataa actccgcgtt gaaaagctca gtagacgaag gaatcaaatc  1800
   caggttaggc cagtaagtct cctgaggaag ccctcgcata tcgaaggact cgcccgccaa  1860
   atacgcctcg atgagcggca taatggtgtt ttcggacgac acctcggcat gtggattgat   1920
   gccatacagc gtggttgcgc ttccctgcgg atccagatca acgtggcaca ccttccgacc   1980
   tcgccgcaga ctcaagccct ggcagaggag ggtggacata gtggtcttcg tgacaccgcc   2040
   cttgaagttc gcaactgcaa tgactttacc tggtcccctt cgcgggacag gcttaagttc   2100
   cgctcggatc cattgcattg cctcgctgag gctgaaatag cgcactgcac cgggtcgcga   2160
   ttgctggccc tgagggagat cccccttttt gagggaccgg ttcatccgcg ttttgtcgat   2220
   gccacacaat tcttgtaggc ggccagacgg gatcagcggg gcttccttcc gaggaaatgg   2280
   ctccagcatc tcatcccgaa ttttctgcag catcaccgcg gagagatttg ccaactgctt   2340
   caaatcctca ggcgtcgtct tgctggggtg ggctagcttg atatccattc ggttcaaaaa   2400
   cctttaagct gttcaccgga accgccggtg gatcgtgcaa ccgttttcgg caaaaatgac   2460
   gtccgcggtt gcgttgccgt gaaggttagc ggcccttaga cgtatacgca agttaacttt   2520
   gtgcctgttg tacccgagag atcgagctgt tcccccggta cttacgggtc gttgttaaag   2580
   taaaaggcga ggaattctac gaaagtgcca tataacgcac aaagaatgcg cgcctcagga   2640
   agtaaggctt aggccagcct caaggacgaa gcgcgaggtc gggttcttct tcgccgttga   2700
   gataaacagg caatccctgg cgcgcgtgac ggcgacgtag aagaggcgcc tttcttcgct   2760
   ttctgggctt tcgctgtcgg gaacgattgt ctcttcggct cggactatgg ctacgctgtc   2820
   ccactcccgt ccttttgagg cgtgtaca                                      2848
<210> 9
   <211> 26
   <212> DNA
   <213> Artificial
<220>
   <223> Primer
<400> 9
   aggttctggc cgccggactc cagggt 26
<210> 10
   <211> 32
   <212> DNA
   <213> Artificial
<220>
   <223> Primer
<400> 10
   ggagagcata tgagccaacc atcttatggc cc 32
<210> 11
   <211> 26
   <212> DNA
   <213> Artificial
<220>
   <223> Primer
<400> 11
   accctggagt ccggcggcca gaacct 26
<210> 12
   <211> 55
   <212> DNA
   <213> Artificial
<220>
   <223> Primer
<400> 12
   atggatcctg cagttagtgg tggtggtggt ggtgtgcggc gtcctcctct gttgg 55
<210> 13
   <211> 2377
   <212> DNA
   <213> Artificial
<220>
   <223> PHAsynthase
<400> 13
   gaattccgat ctggaccggg gtgctggcct gggccacgcc ggcgagggcc agcgcggagc     60
   aaccgagcag cagggcgaga ggtttcatcg ggattccttg gcagtctgaa tgacgtgcca    120
   gcctatcagc gcggcgccgg tgcggcgagg gcgcgccgga cccagtgcgt cacctctcgt    180
   ctgatccgcc tccctcgacg ggcgtcgctg acaaaaaaat tcaaacagaa attaacattt    240
   atgtcattta caccaaaccg catttggttg cagaatgctc aaacgtgtgt ttgaacagag    300
   caagcaacac gtaaacaggg atgacatgca gtacccgtaa gaagggccga ttggcccaga    360
   tctcgcctcg ggtgtgggtg aaggagagca catgagccaa ccatcttatg gcccgctgtt    420
   cgaggccctg gcccactaca atgacaagct gctggccatg gccaaggccc agacagagcg    480
   caccgcccag gcgctgctgc agaccaatct ggacgatctg ggccaggtgc tggagcaggg    540
   cagccagcaa ccctggcagc tgatccaggc ccagatgaac tggtggcagg atcagctcaa    600
   gctgatgcag cacaccctgc tcaaaagcgc aggccagccg agcgagccgg tgatcacccc    660
   ggagcgcagc gatcgccgct tcaaggccga ggcctggagc gaacaaccca tctatgacta    720
   cctcaagcag tcctacctgc tcaccgccag gcacctgctg gcctcggtgg atgccctgga    780
   gggcgtcccc cagaagagcc gggagcggct gcgtttcttc acccgccagt acgtcagcgc    840
   catggccccc agcaacttcc tggccaccaa ccccgagctg ctcaagctga ccctggagtc    900
   cggcggccag aacctggtgc gcggactggc cctcttggcc gaggatctgg agcgcagcgc    960
   cgatcagctc aacatccgcc tgaccgacga atccgccttc gagctcgggc gggatctggc   1020
   cctgaccccg ggccgggtgg tgcagcgcac cgagctctat gagctcattc agtacagccc   1080
   gactaccgag acggtgggca agacacctgt gctgatagtg ccgcccttca tcaacaagta   1140
   ctacatcatg gacatgcggc cccagaactc cctggtcgcc tggctggtcg cccagggcca   1200
   gacggtattc atgatctcct ggcgcaaccc gggcgtggcc caggcccaaa tcgatctcga   1260
   cgactacgtg gtggatggcg tcatcgccgc cctggacggc gtggaggcgg ccaccggcga   1320
   gcgggaggtg cacggcatcg gctactgcat cggcggcacc gccctgtcgc tcgccatggg 1380
   ctggctggcg gcgcggcgcc agaagcagcg ggtgcgcacc gccaccctgt tcactaccct 1440
   gctggacttc tcccagcccg gggagcttgg catcttcatc cacgagccca tcatagcggc 1500
   gctcgaggcg caaaatgagg ccaagggcat catggacggg cgccagctgg cggtctcctt 1560
   cagcctgctg cgggagaaca gcctctactg gaactactac atcgacagct acctcaaggg 1620
   tcagagcccg gtggccttcg atctgctgca ctggaacagc gacagcacca atgtggcggg 1680
   caagacccac aacagcctgc tgcgccgtct ctacctggag aaccagctgg tgaaggggga 1740
   gctcaagatc cgcaacaccc gcatcgatct cggcaaggtg aagacccctg tgctgctggt 1800
   gtcggcggtg gacgatcaca tcgccctctg gcagggcacc tggcagggca tgaagctgtt 1860
   tggcggggag cagcgcttcc tcctggcgga gtccggccac atcgccggca tcatcaaccc 1920
   gccggccgcc aacaagtacg gcttctggca caacggggcc gaggccgaga gcccggagag 1980
   ctggctggca ggggcgacgc accagggcgg ctcctggtgg cccgagatga tgggctttat 2040
   ccagaaccgt gacgaagggt cagagcccgt ccccgcgcgg gtcccggagg aagggctggc 2100
   ccccgccccc ggccactatg tcaaggtgcg gctcaacccc gtgtttgcct gcccaacaga 2160
   ggaggacgcc gcatgagcgc aggatccgtg gtcaagctgc cttaagcacc ggcggcacgc 2220
   aggcacaatc agcccggccc ctgccgggct gattgttctc ccccgctccg cttgccccct 2280
   ttttcggggc aatttggccc aggccctttc cctgccccgc ctaactgcct aaaatggccg 2340
   ccctgccgtg taggcattca tccagctaga ggaattc 2377
<210> 14
   <211> 933
   <212> DNA
   <213> Artificial
<220>
   <223> Kanamycin resistance gene
<400> 14
   ccggaattgc cagctggggc gccctctggt aaggttggga agccctgcaa agtaaactgg      60
   atggctttct cgccgccaag gatctgatgg cgcaggggat caagctctga tcaagagaca     120
   ggatgaggat cgtttcgcat gattgaacaa gatggattgc acgcaggttc tccggccgct     180
   tgggtggaga ggctattcgg ctatgactgg gcacaacaga caatcggctg ctctgatgcc     240
   gccgtgttcc ggctgtcagc gcaggggcgc ccggttcttt ttgtcaagac cgacctgtcc     300
   ggtgccctga atgaactgca agacgaggca gcgcggctat cgtggctggc cacgacgggc     360
   gttccttgcg cagctgtgct cgacgttgtc actgaagcgg gaagggactg gctgctattg     420
   ggcgaagtgc cggggcagga tctcctgtca tctcaccttg ctcctgccga gaaagtatcc     480
   atcatggctg atgcaatgcg gcggctgcat acgcttgatc cggctacctg cccattcgac     540
   caccaagcga aacatcgcat cgagcgagca cgtactcgga tggaagccgg tcttgtcgat     600
   caggatgatc tggacgaaga gcatcagggg ctcgcgccag ccgaactgtt cgccaggctc     660
   aaggcgagca tgcccgacgg cgaggatctc gtcgtgaccc atggcgatgc ctgcttgccg     720
   aatatcatgg tggaaaatgg ccgcttttct ggattcatcg actgtggccg gctgggtgtg     780
   gcggaccgct atcaggacat agcgttggct acccgtgata ttgctgaaga gcttggcggc     840
   gaatgggctg accgcttcct cgtgctttac ggtatcgccg ctcccgattc gcagcgcatc     900
   gccttctatc gccttcttga cgagttcttc tga                                  933
<210> 15
   <211> 75
   <212> DNA
   <213> Artificial
<220>
   <223> Primer
<400> 15
   tggcccagat ctcgcctcgg gtgtgggtga aggagagcac atgagcgcac aatccctgga      60
   agtaggccag aaggc                                                       75
<210> 16
   <211> 78
   <212> DNA
   <213> Artificial
<220>
   <223> Primer
<400> 16
   gccggtgctt aaggcagctt gaccacggat cctgcgtcat taaggcagct tgaccacggc      60
   ttcccccgtc acggcgag                                                    78
<210> 17
   <211> 453
   <212> DNA
   <213> Artificial
<220>
   <223> (R)-specific enoyl-coA hydratase
<400> 17
   tggcccagat ctcgcctcgg gtgtgggtga aggagagcac atgagcgcac aatccctgga     60
   agtaggccag aaggcccgtc tcagcaagcg gttcggggcg gcggaggtag ccgccttcgc    120
   cgcgctctcg gaggacttca accccctgca cctggacccg gccttcgccg ccaccacggc    180
   gttcgagcgg cccatagtcc acggcatgct gctcgccagc ctcttctccg ggctgctggg    240
   ccagcagttg ccgggcaagg ggagcatcta tctgggtcaa agcctcagct tcaagctgcc    300
   ggtctttgtc ggggacgagg tgacggccga ggtggaggtg accgcccttc gcgaggacaa    360
   gcccatcgcc accctgacca cccgcatctt cacccaaggc ggcgccctcg ccgtgacggg    420
   ggaagccgtg gtcaagctgc cttaagcacc ggc                                 453
<210> 18
   <211> 91
   <212> DNA
   <213> Cupriavidus metallidurans
<400> 18
   ctctcccatg tcgcgcttac gacatctgag agaaagtggc cttgctcttt agcctttcgc     60
   atcaaccaaa aaattaaatt tacatcaaga c                                    91
<210> 19
   <211> 2291
   <212> DNA
   <213> Cupriavidus metallidurans
<400> 19
   caaaagccca cggttgccgc cgtgggcttt tgtttttgta ggattacagt ctagacatgg      60
   cttaaaacct cgattcgtag tacgaagtcc ttaaaaaaca agcacttagc tttttgcgtc     120
   ttccgacgca gctttagcta acccttccag atgttcgcga atggccgatt gggccgcttc     180
   cgcttcttcc tcgctgcgga actcaatgcg catgactttc tttgcgatac gaacatcgca     240
   ccaagtcgcc tttcccgcct tgattttgaa gccggtagat gcggcaggcc tggtcttaac     300
   ctgctcggcc ttagtcatcc gtacggcctg ctgttgatcg atcttcttct cggccaacaa     360
   cttaaccgct tcgacgaccc gatccccgcg accgtctttg gtcgcccttg ccaactcggc     420
   gccagcattc gacccgagca ggtctgggcg gctatcgaga aggcttagga cctcgggagg     480
   caagttccgg aacgacaaaa tctctgagag agtcgagaca ggtacaccag cctgttcagc     540
   tattgcggct tgagtcttgt ctgggctgcg aagcagcagt tcgtcgaact tccgaaactt     600
   ctcgaaatcc gttaaatctg actgcataag gttcgcgtag aacgcgcccg tgtcagcctc     660
   gtctgaacta agttcgccga gcacgcagcg tatgtgatcg cgcccaagct cgcggtacgc     720
   gtctgtccgg tgatgcccgg agacaatctc gaagcctccc gcaggccgag ggcatacaat     780
   cacaggatga acgagcttgt tgtgcctgag gttttccctc aattcgacat acttctcggg     840
   aggcatgaag cgtctgcggc ctgggacctc atgcagttgg tcgataggca catccactgc     900
   gccgccgctg acgcgggccg actcaagttc tgctcttagt tgcgcgatct cgttagcctg     960
   tcgctcaacc ttcccttgca tatgcattaa ctgaccgggc gcagttcgcg gcgcttgcgg    1020
   accgcgcgca gcggctttct ccaagtcttc cgccgtgacc ttgatgttcc cagcctttgc    1080
   ggcaagcttt tctctcaaac tcattcttca attccttgcc aaagcgcgct gaccttgtcg    1140
   tccacatagt cgacaaactc atcgcagggt tggcgaatgc gtcgcatcgt ctcggtgttc    1200
   gcctcgctag aggagaggtc atagaccgtg cgaaattgaa tgctgctgtt acgggcgagg    1260
   tccgtctcag ggatctcaat cggcagcacg cgcgacccat agacgccgcg aatccagtct    1320
   gccaccaggc gaggagcgtt ctttttctcc atgcgtgtca tgagaacatc gagaaagtcg    1380
   aactcctttt tagatccctc gctctgctct tccatgcctg ttactaggtc cgagaagagt    1440
   tgccagaact ggaccataga cgcgaaagcc aaggtgtccg ggaccaccgg tacgatgacg    1500
   ccatcggcag cgaaaatcgc gttgatggtc aggtagctga gggtaggagc cgtgtcgagg    1560
   atgatgtagt catattcgtc tttcaacgaa tcgaggccgt tacttaacac gcgctcgaac    1620
   ggaatatggc cttcctctgc cgtcgcccga gccggaagca taaactccgc gttgaaaagc 1680
   tcagtagacg aaggaatcaa atccaggtta ggccagtaag tctcctgagg aagccctcgc 1740
   atatcgaagg actcgcccgc caaatacgcc tcgatgagcg gcataatggt gttttcggac 1800
   gacacctcgg catgtggatt gatgccatac agcgtggttg cgcttccctg cggatccaga 1860
   tcaacgtggc acaccttccg acctcgccgc agactcaagc cctggcagag gagggtggac 1920
   atagtggtct tcgtgacacc gcccttgaag ttcgcaactg caatgacttt acctggtccc 1980
   cttcgcggga caggcttaag ttccgctcgg atccattgca ttgcctcgct gaggctgaaa 2040
   tagcgcactg caccgggtcg cgattgctgg ccctgaggga gatccccctt tttgagggac 2100
   cggttcatcc gcgttttgtc gatgccacac aattcttgta ggcggccaga cgggatcagc 2160
   ggggcttcct tccgaggaaa tggctccagc atctcatccc gaattttctg cagcatcacc 2220
   gcggagagat ttgccaactg cttcaaatcc tcaggcgtcg tcttgctggg gtgggctagc 2280
   ttgatatcca t 2291

## Description

### TECHNICAL FIELD

The present invention relates to a transformant comprising a vector as defined in the claims. The invention also relates to the field of plasmid stabilization and, more particularly, it relates to a recombinant vector capable of being stably retained in bacteria of the species *Cupriavidus necator,* which are hydrogen bacteria and are known to be PHB-synthesizing bacteria, to a bacterial strain that belongs to the species *Cupriavidus necator* transformed with that vector, and to commercial production of polyhydroxyalkanoates using that bacterial strain.

### BACKGROUND ART

When actually applied to the production of a certain desired substance by microorganisms, the recombinant DNA technology generally presents a problem that the recombinant plasmid is unstable.

The cloning and expression vectors generally used in laboratories are mostly multicopy plasmids, and the stable transmission thereof to later generations can be secured by introduction of a number of plasmids per cellular genome (cf. Non Patent Document 1). However, when a foreign gene is introduced using a plasmid, plasmid elimination occurs during bacterial proliferation cycles, resulting in the instability of the gene introduced. Therefore, in a commercial production process, it is essential to stabilize the plasmid in bacteria until completion of the culture in a fermentor.

So far pJRD215-derived vectors and pBBR-derived vectors have been mainly used as plasmid vectors for gene transfer into bacteria of the genus *Ralstonia, Wautersia* and *Cupriavidus* (Non Patent Document 2 and 3). However, investigations made by the present inventors have revealed that these plasmid vectors become very unstable upon accumulation of polyhydroxyalkanoates, among others, in cells by host bacteria. In the case of a pJRD215-derived vector, for instance, about 80% of the cells after 4 passages under cultivation conditions under which the accumulation of polyhydroxyalkanoates is not rich, without application of any antibiotic-due selective pressure, retain the plasmid, whereas, after 4 passages under cultivation similarly conditions allowing a great accumulation of the polyesters, only 30% of the cells retain the plasmid. The same feature is presented by pBBR-derived vectors.

These plasmid vectors are broad-host-range vectors developed for use in a wide range of hosts (cf. Non Patent Document 4 and 5). However, in commercial substance productions using bacteria of the genus *Ralstonia, Wautersia* or *Cupriavidus,* for instance, as the hosts, it is necessary to develop plasmid vectors for gene transfer suited for use in bacteria of the genus *Ralstonia, Wautersia* or *Cupriavidus.*

Various techniques have so far been developed to stabilize plasmids. However, as regards plasmid vectors capable of being used in the genus *Ralstonia,* it is only possible to select plasmid-retaining transformants according to the resistance to such a drug as chloramphenicol, kanamycin or ampicillin. When the cultivation is carried out under application of an antibiotic-due selective pressure, the following problems arise: (1) the use of an antibiotic-resistant strain may possibly involve risk to the environment, (2) the amount of an antibiotic which is required during cultivation significantly increases the cost of production, and (3) the use of antibiotics is undesirable for the production of substances to be used in the treatment of humans and animals. Thus, transformants retaining a plasmid owing to drug resistance cannot be applied to commercial production.

In addition to the technique comprising applying an antibiotic-due selective pressure, the so-called par system is known as a system for stable retention of a plasmid (cf. Non Patent Document 6 and 7). When the par system works, the plasmids replicated are partitioned among daughter cells, so that a bacterial strain stably retaining a plasmid can be obtained without applying any selective pressure due to antibiotic resistance. Thus, a vector with the par system of the RP4 plasmid, which is usable in *Escherichia coli,* inserted therein (cf. Patent Document 1) and a vector in which the par region of the R1 plasmid is utilized (cf. Patent Document 2) have so far been developed. Further, it is also known that, in the par region of the megaplasmid pMOL28 retained in the *Cupriavidus metallidurans* CH34 strain, there occur the promoter parP, the plasmid stabilizing factors parA28 and parB28 and the recognition sequence parS; these genes have already been cloned in the plasmid pSUP202 and the nucleotide sequences thereof have been published (cf. Non Patent Document 8).

As described above, since bacteria belonging to the genus *Ralstonia, Cupriavidus* or *Wautersia* (in particular the species *Cupriavidus necator*) are often used as bacteria for the production of polyhydroxyalkanoates or the production of proteins (cf. Non Patent Document 9 and 10), it is required to develop a plasmid vector which is particularly usable in these bacteria, has no transferability by conjugation, and is capable of being stably retained therein without any antibiotic-due selective pressure. However, as far as bacteria belonging to the genus *Ralstonia, Cupriavidus* or *Wautersia* are concerned, such plasmid stabilization using the par region has not been made as yet; how to construct such system or whether such system is actually effective for plasmid stabilization is unknown.
Patent Document 1: U.S. Patent No. 6,143,518
Patent Document 2: U.S. Patent No. 4,760,022
Non Patent Document 1: Jones IM et al., Mol. Gen. Genet. 180(3): 579-84 (1980)
Non Patent Document 2: T. Fukui et al., Biotechnology Letters, Vol. 19, No. 11, Nov. 1997; 1093-97)
Non Patent Document 3: Steven Slater et al., Journal of Bacteriology, Apr. 1998: 1979-1987
Non Patent Document 4: Luan Tao et al., Metabolic Engineering, Volume 7, Issue 1, January 2005: 10-17
Non Patent Document 5: Davison J et al., Gene, 1987; 51(2-3): 275-80
Non Patent Document 6: M. Gerlitz et al., Journal of Bacteriology, Nov.: 6194-6203 (1990)
Non Patent Document 7: B. Youngren et al., Journal of Bacteriology, July: 3924-3928 (2000)
Non Patent Document 8: Safieh Taghavi et al., Mol. Gen. Genet. 250: 169-179 (1996)
Non Patent Document 9: Gravin C. et al., Protein Expression & Purification Dec; 38(2): 64-71 (2004)
Non Patent Document 10: Jackson J.K. et al., Journal of Biotechnology, 1999; 68:49-60.

### SUMMARY OF THE INVENTION

It is an object of the present invention to develop a transformant comprising a vector. More particularly, the object is to develop a vector which can be used in bacteria of the species *Cupriavidus necator* as the hosts and can be stably retained therein without any antibiotic-due selective pressure. Another object is to cause the resulting transformant to produce a polyhydroxyalkanoate and, further, stabilize the productivity.

### DETAILED DESCRIPTION OF THE INVENTION

In constructing a vector capable of being used for transforming desired bacteria of the species *Cupriavidus necator*, among others, a DNA sequence (ori) is essential for vector replication in host bacteria. Therefore, the present inventors conjectured that by developing a vector which contains an origin of DNA replication (ori) functioning in bacteria of the species *Cupriavidus necator,* for instance, it might become possible to construct a vector which can be used in bacteria of the species *Cupriavidus necator* as the hosts. The vector to be used for the commercial substance productions is essential to have (1) DNA base pairs the number of which is convenient for handling, (2) no drug-resistance gene for transformant selection, (3) no transferability by conjugation, and the like properties in addition to the property that the vector can be replicated inside host bacteria.

Further, the above-mentioned par system is considered to be very useful as a means for vector stabilization for the reason that antibiotic addition is unnecessary and it is not necessary to introduce any mutation into the chromosome of host bacteria, among others. However, although the above-mentioned megaplasmid pMOL28 can be retained in host bacteria because of the par system thereof, this megaplasmid has such a large number of base pairs as 280 k and no drug-resistance gene. For this reason, this megaplasmid has not been thought to be usable as a vector for bacteria of the species *Cupriavidus* necator.

Accordingly, the present inventors made intensive investigations in an attempt to solve the above subjects and, as a result, succeeded in developing a plasmid vector from the finding that a vector can be stably retained without application of an antibiotic-due selective pressure by causing the origin of replication and the par system of the megaplasmid pMOL28 retained by the *Cupriavidus metallidurans* CH34 strain to function in the species *Cupriavidus necator* (former name: *Ralstonia eutropha,* or *Wautersia eutropha*).

Thus, in a first aspect thereof, the present invention relates to a transformant which is obtained from gene transfer into a host bacterium by means of a recombinant vector which contains an origin of replication as introduced therein, wherein the origin of replication contains the sequence identified under SEQ ID NO:18 and functions in bacteria of the species *Cupriavidus necator* as hosts. The recombinant vector has neither the mob gene nor the oriT sequence, namely has no transferability by conjugation, is obtained from introduction of the par region, which is a DNA region functioning as the par system, namely as a plasmid-stabilizing mechanism and contains, as introduced therein, at least one gene involved in the PHA synthesis as selected from the group of genes consisting of the genes for thiolase and reductase constituting a system for providing 3-hydroxybutyric acid (3HB), the gene for polyhydroxybutyrate (PHB) synthase, namely PHB synthase, the gene for polyhydroxyalkanoate (PHA) synthase, namely PHA synthase, and the genes for acyl-CoA transferase, enoyl-CoA hydratase and acyl-CoA dehydrogenase, which are enzymes in the β oxidation pathway. Still more preferably the recombinant vector contains the DNA fragment identified under SEQ ID NO: 19 as the par region.

The transformant of the invention is derived from a host cell belonging to the species *Cupriavidus necator*.

In a second aspect, the invention relates to a method for producing a PHA which comprises cultivating the transformant mentioned above and recovering the PHA from the culture.

In the following, the invention is described in detail.

The recombinant vector according to the first aspect of the invention contains an origin of replication as introduced therein, wherein the origin of replication contains the sequence identified under SEQ ID NO:18 and functions in bacteria of the species *Cupriavidus necator* as hosts.

The origin of replication is a sequence functioning as the site of origin of replication of the recombinant vector. The sequence shown under SEQ ID NO:18 is a part of the origin of replication (ori region) shown under SEQ ID NO:7 in the megaplasmid pMOL28 retained by the *Cupriavidus metallidurans* CH34 strain. The origin of replication to be introduced into a vector in accordance with the invention may be any one containing the sequence identified under SEQ ID NO:18 and capable of functioning in bacteria of the species *Cupriavidus necator* as hosts. The sequence shown under SEQ ID NO:7 may be used as the above-mentioned origin of replication.

The recombinant vector of the transformant of the invention is free of the mob gene group and of the oriT sequence. If the vector has the mob gene group and/or the oriT sequence, transfer by conjugation may occur upon contacting with another microorganism, posing a problem from the safety viewpoint, namely from the viewpoint of transformant containment. Here, the mob gene group refers to a group of genes coding for DNA transferring functions, and the proteins encoded by the mob gene group have a function in making a nick in the oriT sequence and, further, a function in stably transferring a DNA rendered single-stranded. The oriT sequence comprises a nick site and a recognition sequence for nicking.

The recombinant vector of the transformant of the invention is one resulting from introduction of a vector stabilizing region (par region) functioning as the par system. The par region may be any sequence that can serve as the par system functioning in bacteria of the species *Cupriavidus necator*. Preferred is, however, the par region in a plasmid retained by a bacterial strain belonging to the genus *Ralstonia*, *Cupriavidus* or *Wautersia* and, more preferably, the par region in the megaplasmid. In the practice of the invention, a DNA fragment containing the sequence defined under SEQ ID NO: 19 is most preferably used. The sequence shown under SEQ ID NO:19 is a part of the par region shown under SEQ ID NO:8 in the megaplasmid pMOL28 retained by the *Cupriavidus metallidurans* CH34 strain and contains the parA gene, parB gene and recognition sequence pars.

Since the sequence shown under SEQ ID NO:19 is a part of the par region in the megaplasmid pMOL28 retained by the *Cupriavidus metallidurans* CH34 strain, it is necessary for the desired vector to contain a promoter and a terminator in addition to the sequence shown under SEQ ID NO:19 so that the DNA fragment containing that sequence may function as the par system.

As the promoter, use may be made of the parP in the megaplasmid pMOL28 or a promoter derived from another organism but capable of functioning in bacteria of the species *Cupriavidus* necator as hosts. The parP promoter in the megaplasmid pMOL28 is almost identical with the base sequence from the 2388th to 2848th nucleotide in the sequence shown under SEQ ID NO:8.

As the terminator, use may be made of the terminator in the megaplasmid pMOL28 or a terminator derived from another organism but capable of functioning in bacteria of the species *Cupriavidus necator* as hosts. The terminator in the megaplasmid pMOL28 is contained in the base sequence from the 61st to the 202nd nucleotide in the sequence shown under SEQ ID NO:8.

The sequence shown under SEQ ID NO: 8 may also be used as the par region to be introduced into the recombinant vector of the transformant of the invention.

The recombinant vector of the transformant of the invention contains at least one gene involved in the PHA synthesis as introduced therein. Then, the transformant retaining the recombinant vector will be able to efficiently synthesize a PHA if all the genes concerned function effectively. And, in this transformant capable of synthesizing a PHA, unlike multicopy plasmids, the plasmid replicates stably, so that it is possible to stably feed the genes involved in PHA synthesis to hosts for the accumulation of the PHA in commercially significant amounts.

The gene involved in PHA synthesis includes the genes for thiolase and reductase constituting a 3HB supplying system, the gene for a PHB synthesizing enzyme, namely PHB synthase, the gene for a PHA synthesizing enzyme, namely PHA synthase, and the genes for acyl-CoA transferase, enoyl-CoA hydratase and acyl-CoA dehydrogenase, which are enzymes in the β oxidation pathway. The recombinant vector contains at least one member selected from among those mentioned above as introduced therein. The thiolase is, for example, β-ketothiolase; the reductase is, for example, acetoacetyl-CoA reductase; the PHA synthase is, for example, *Aeromonas caviae*-derived PHA synthase mutant gene, namely N149S/D171G; and the acyl-CoA transferase is, for example, 3-hydroxyacyl-ACP-CoA transferase.

When, in introducing the gene or genes involved in the PHA synthesis into the recombinant vector of the transformant of the invention, a restriction site or sites are introduced into the vector in advance, it becomes easy to introduce the gene(s) in question.

Further, the recombinant vector of the transformant of the invention preferably contains a selective marker. While the introduction of the par region into the recombinant vector in accordance with the invention provides the vector with excellent stability and, therefore, no selective pressure due to antibiotic etc. is required, as described later herein, the above-mentioned selective marker can be utilized in transformant selection following transformation of host cells with the recombinant vector of the transformant of the invention.

The selective marker is not particularly restricted but maybe, for example, the kanamycin-, ampicillin-, tetracycline-or like antibiotic-resistance gene. In the recombinant vector of the transformant of the invention, the kanamycin-resistance gene shown under SEQ ID NO:14 is preferred as the selective marker.

The recombinant vector of the transformant of the invention may also be a smaller version derived from the recombinant vector obtained after introduction of the above-mentioned gene or genes by rendering the same small-sized.

The recombinant vector can be made small-sized by deleting those portions which are unnecessary for the expression of the origin of replication, the par region, the selective marker and the PHA synthase genes. For example, since the recombinant vector of the tranformant of the invention is free of the mob gene group and the oriT sequence, the size reduction can be realized by deleting such gene group and sequence. When rendered small-sized, the recombinant vector of the transformant of the invention can be introduced into hosts at an improved transformation ratio.

The recombinant vector of the transformant of the invention more preferably contains an origin of replication, wherein the origin of replication contains the sequence identified under SEQ ID NO:18 and functions in bacteria of the species *Cupriavidus necator* as hosts and the kanamycin-resistance gene shown under SEQ ID NO:14.

The recombinant vector of the transformant of the invention, which contains the megaplasmid-derived origin of replication, becomes a recombinant vector capable of functioning also in bacteria of the species *Cupriavidus necator* as hosts.

The vector to be used in constructing the recombinant vector of the transformant of the invention is not particularly restricted but various plasmids and phages, among others, can be used. In view of the possibility of the resulting recombinant vector serving as a shuttle vector that can replicate in *Escherichia coli* as well, however, the use of an *Escherichia coli*-derived plasmid is preferred.

The method of constructing the recombinant vector of the tranformant of the invention is not particularly restricted but the desired vector can be constructed from any plasmid vector by inserting thereinto the origin of replication functioning in bacteria of the species *Cupriavidus necator* as well as a selective marker employed according to need, such as the antibiotic resistance imparting gene (e.g. kanamycin-, ampicillin- or tetracycline-resistance gene) and the par region functioning as the recombinant vector stabilizing par system.

The transformant according to the first aspect of the invention is one obtained by transformation with the above-mentioned recombinant vector. Namely, the transformant of the invention is obtained by introducing the recombinant vector obtained in the above manner into a host bacterial cell displaying competence to the vector.

The host to be used in the practice of the invention is of the species *Cupriavidus necator*. This species *Cupriavidus necator* is taxonomically identical with *Ralstonia eutropha* and with *Wautersia eutropha* [Vaneechoutte M. et al. , Int. J. Syst. Evol. Microbiol., Mar. 54 (Pt 2) : 317-327 (2004) ; Vadamme P. et al., Int. J. Syst. Evol. Microbiol., Nov. 54 (Pt 6): 2285-2589 (2004)].

The transformant of the invention retains the ori region and a part of the par region of the megaplasmid pMOL28 retained by the *Cupriavidus metallidurans* CH34 strain as introduced thereinto by means of the above-mentioned recombinant vector. Generally, upon introduction into a microorganism retaining a megaplasmid of a gene derived from the megaplasmid, homologous recombination may occur or the introduction of the plasmid containing the same gene may be rejected, so that it is difficult to further introduce a gene in the megaplasmid originally retained by the microorganism into the microorganism. When hosts of the species *Cupriavidus necator* are transformed using the above-mentioned recombinant vector in accordance with the invention, however, very high replication ability and stability can be attained presumably due to non-occurrence of competition between the origin of replication in the megaplasmid originally retained by the host and the origin of replication introduced by the recombinant vector, among others, and due to synergy between the replication abilities and stability effects of both.

The method for producing the transformant of the invention is not particularly restricted but the introduction of the recombinant vector into a host can be carried out by any of the methods known in the art. For example, use can be made of the electroporation method (Current Protocols in Molecular Biology, Vol. 1, page 1.8.4, 1994) and the calcium method (Lederberg, E. M. et al., J. Bacteriol., 119, 1072 (1974)). In transformant selection, the selective marker such as the kanamycin resistance expression system etc. can be used. In the practice of the invention, microorganisms of the species *Cupriavidus necator* are used as hosts, so that the preliminary procedure for eliminating the megaplasmid from the hosts is unnecessary.

Now, the method of PHA production according to the invention is described.

The PHA to be produced according to the invention is represented by the following general formula (1): 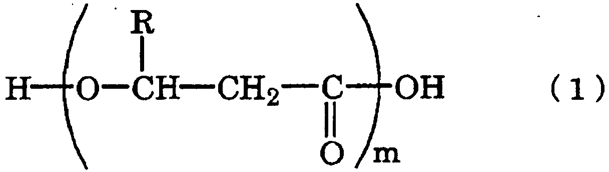 (in the above formula, R represents an alkyl group containing 1 to 13 carbon atoms and m represents an integer of not smaller than 2. The m R groups may be the same or different).

Preferred as the above PHA is the copolyester P(3HB-co-3HH) constituted of 3-hydroxybutyric acid and 3-hydroxyhexanoic acid monomer units as represented by the general formula (2): 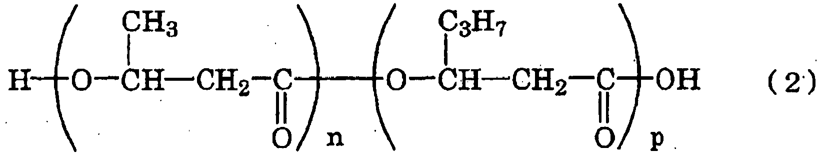 (in the above formula, n and p each represents an integer of not smaller than 1).

The method of PHA production according to the invention comprises cultivating the transformant mentioned above and extracting and purifying the PHA from the culture. The method referred to just above is not particularly restricted but can be carried out in the following manner.

In the polyhydroxyalkanoate production, the above-mentioned transformant can be cultured using a medium containing such carbon sources as sugar, oil and fat, and/or a fatty acid as well as nutrient sources other than carbon sources, namely a nitrogen source, an inorganic salt and one or more other organic nutrient sources. Thus, for example, a medium containing a carbon source assimilable by the microorganism, optionally with at least one of the nitrogen source, inorganic salt and organic nutrient sources being restricted, for example with the nitrogen source being restricted to 0.01 to 0.1%, can be used as the medium for cultivating the transformant obtained from a host microorganism belonging to the species *Cupriavidus necator.*

As the sugar, there may be mentioned, for example, carbohydrates such as glucose and fructose. As the oil and fat, there may be mentioned oils and fats containing a large amount of saturated and/or unsaturated fatty acids having 10 or more carbon atoms, for example, coconut oil, palm oil, palm kernel oil, and the like. As the fatty acid, there may be mentioned saturated and/or unsaturated fatty acids such as hexanoic acid, octanoic acid, decanoic acid, lauric acid, oleic acid, palmitic acid, linoleic acid, linolenic acid and myristic acid, or derivatives, such as esters and salts, of these fatty acids.

As the nitrogen source, there may be mentioned, for example, ammonia, ammonium salts such as ammonium chloride, ammonium sulfate and ammonium phosphate, peptone, meat extract, yeast extract, and the like. As the inorganic salt, there may be mentioned, for example, potassium dihydrogenphosphate, dipotassium hydrogenphosphate, magnesium phosphate, magnesium sulfate, sodium chloride, and the like. As the other organic nutrient source, there may be mentioned, for example, amino acids such as glycine, alanine, serine, threonine and proline; vitamins such as vitamin B1, vitamin B12 and vitamin C; and the like. Additionally, antibiotics (kanamycin, etc.), which corresponds to the drug-resistance gene existing in the expression vector, may be added into the culture fluid.

The culture may be carried out at any temperature as long as the cells can grow, and preferably at 20 to 40°C. The culture period is not particularly restricted, and may be about 1 to 10 days. Then, PHAs can be recovered from the obtained culture cells.

In the present invention, the PHA recovery from the cells may be carried out by the following methods, for example. After completion of the culture, the cells are separated from the culture fluid using a centrifugal machine, etc., washed with distilled water, methanol, etc., and then dried. From these dried cells, PHAs are extracted using an organic solvent such as chloroform. From the PHA-containing organic solvent solution, cell components are removed by filtration, etc., and a poor solvent such as methanol or hexane is added to the filtrate to precipitate PHAs. Then, the supernatant fluid is removed by filtration or centrifugation, and the obtained PHAs are dried. In such manner, PHAs are recovered.

Analysis of weight average molecular weight (Mw) and 3HH composition (mol%) of the obtained PHAs can be carried out by the gas chromatograph, nuclear magnetic resonance, and the like methods, for example. Alternatively, as a simple method for confirming production of PHAs, a staining method using Nile red can be used. That is, whether polyesters are produced can be confirmed by a method comprising adding Nile red to an agar medium in which recombinant cells grow, culturing the recombinant cells for 1 to 7 days, and observing whether the recombinant cells turn red or not.

The recombinant vector of the transformant of the invention can be used in bacteria of the species *Cupriavidus necator* as hosts. As it contains neither the mob gene group nor the oriT sequence, in particular, it has no transferability by conjugation and, further, as it contains the par region introduced therein, it can be stably retained in bacteria without any antibiotic-due selective pressure. Transformants obtained by using that vector can stably produce a PHA.

### BEST MODES FOR CARRYING OUT THE INVENTION

The following examples illustrate the present invention more specifically. These examples are, however, by no means limitative of the scope of the invention. The general gene manipulation procedures can be carried out as described in Molecular Cloning (Cold Spring Harbor Laboratory Press (1989)).

The enzymes, cloning hosts and other materials to be used in gene manipulation can be purchased from suppliers on the market and can be used according to the instructions given by the suppliers. As for the enzymes, those which can be used in gene manipulation can be used without any particular restriction.

In the following examples, an *Escherichia coli*-derived plasmid was used; this plasmid contains the kanamycin-resistance gene shown under SEQ ID NO:14.

### (Example 1) Construction of plasmid vector pCUP

The plasmid vector for the introduction of an origin of replication and the par region thereinto in this example is not particularly restricted but may be any one capable of being used in bacteria of the species *Cupriavidus necator.* In the plasmid vector constructed in this example, the origin of replication (SEQ ID NO:7) and the par region given under SEQ ID NO:8 of the megaplasmid (pMOL28) retained by the *Cupriavidus metallidurans* CH34 strain were used. As for the specific construction procedure, a megaplasmid-containing DNA fraction was first prepared from the *Cupriavidus metallidurans* CH34 strain using a DNA purification kit (product of Promega) and, using this DNA fraction as a template and the primers shown under SEQ ID NO:1 and SEQ ID NO:2, a DNA region (about 4 kbp in size) comprising the sequences shown under SEQ ID NO:7 and SEQ ID NO:8 was amplified by the PCR technique. The PCR conditions were: (1) 2 minutes at 98°C; (2) 30 seconds at 98°C, (3) 30 seconds at 55°C, (4) 5 minutes at 72°C, 30 cycles from (2) to (4); and 5 minutes at 72°C. The polymerase used was TaKaRa Pyrobest DNA Polymerase (product of Takara Bio Inc.). The fragment amplified was cloned in the cloning vector PCR-Blunt 2-TOPO (product of Invitrogen Corporation) for *Escherichia coli.*

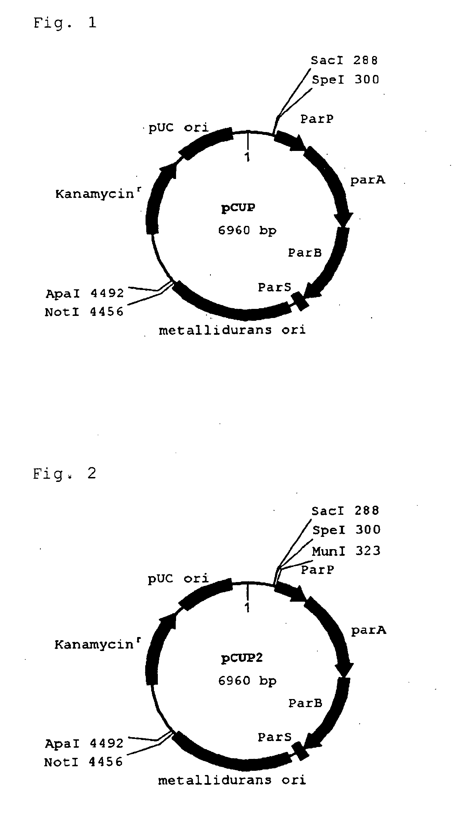

Then, using the primers shown under SEQ ID NO:3 and SEQ ID NO:4, the amplification reaction was allowed to proceed outwardly from both ends of the 2061 bp-2702 bp region of the PCR-Blunt 2-TOPO (product of Invitrogen Corporation) by the PCR technique, followed by ligation using a DNA ligase (Ligation High (product of Toyobo Co.)), to give a vector, pCUP, shown in Fig. 1, resulting from deletion of 641 bp. The PCR conditions were: (1) 2 minutes at 98°C; (2) 30 seconds at 98°C, (3) 30 seconds at 55°C, (4) 7 minutes at 72°C, 30 cycles from (2) to (4); and 7 minutes at 72°C. The polymerase used was TaKaRa Pyrobest DNA Polymerase (product of Takara Bio Inc.).

### (Example 2) Construction of plasmid vector pCUP2

For facilitating gene transfer using the plasmid vector of the transformant of the invention, a restriction enzyme *Mun*I site was further introduced into pCUP obtained in Example 1. As for the specific construction procedure, the PCR was carried out by the PCR technique using pCUP constructed in Example 1 as a template and the primers shown under SEQ ID NO:5 and SEQ ID NO:6, followed by ligation of the amplified fragment using a DNA ligase (Ligation High (product of Toyobo Co.)), whereby pCUP2 shown in Fig. 2 was constructed with a *Mun*I introduced therein. The PCR conditions were: (1) 2 minutes at 98°C; (2) 30 seconds at 98°C, (3) 30 seconds at 55°C and (4) 5 minutes at 72°C, 30 cycles from (2) to (4). The polymerase used was TaKaRa Pyrobest DNA Polymerase (product of Takara Bio Inc.).

Thus was constructed a plasmid vector containing the DNA region identified under SEQ ID NO:18 and the DNA region identified under SEQ ID NO:19 and free of such genes involved in transfer by conjugation as the mob gene group and the oriT sequence.

### (Example 3) Transformant production using plasmid vector pCUP2

The transformation was carried out by the electroporation technique in the following manner. The gene transfer apparatus used for electroporation was the Gene Pulser produced by Bio-Rad Laboratories, Inc., and the cuvette used was BioRad's one with a gap of 0.2 cm. Competent cells (400 µl) of the *Ralstonia eutropha* H16 strain and 5 µl of a plasmid pCUP2 solution prepared were poured into the cuvette, which was then set in the pulser. Electric pulses were applied under the following conditions: electrostatic capacity 25 µF, voltage 1.5 kV, resistance value 800 Q. After pulse application, the bacterial suspension in the cuvette was subjected to 3 hours of shake culture in a nutrient broth medium (product of DIFCO Laboratories) at 30°C, followed by 2 days of cultivation on selective plates (nutrient agar medium (product of DIFCO Laboratories), 100 mg/L of kanamycin) at 30°C, to give a transformant strain.

### (Comparative Example 1) Transformant production using plasmid vector pJRD215

Using the plasmid vector pJRD215 containing neither the sequence shown under SEQ ID NO:18 nor the par region, a transformant strain was obtained in the same manner as in Example 3 except that pJRD215 was used as the plasmid.

### (Example 4) Plasmid retention rate in transformant obtained by using plasmid vector pCUP2

The transformant strain obtained in Example 3 was tested for plasmid retention rate. The test was performed in the following manner. A culture fluid obtained by 24 hours of cultivation of the transformant obtained in Example 3 using the plasmid vector pCUP2 on an MB + meat extract medium comprising 1% (w/v) of meat extract, 1% (w/v) of Bacto-Trypton, 0.2% (w/v) of yeast extract, 0.9% (w/v) of Na₂PO₄/12H₂O and 0.15% (w/v) of KH₂PO₄, pH 6.8, with kanamycin (50 mg/L) added was transferred, in an amount of 1% (v/v), to a kanamycin-free MB + oil medium comprising 1.1% (w/v) of Na₂PO₄·12H₂O, 0.19% (w/v) of KH₂PO₄, 1.29% (w/v) of (NH₄)₂SO₄, 0.1% (w/v) of MgSO₄/7H₂O, 2.5% (w/v) of palm W olein oil and 0.5% (v/v) of a trace metal salt solution (prepared by dissolving 1.6% (w/v) of FeCl₃·6H₂O, 1% (w/v) of CaCl₂·2H₂O, 0.02% (w/v) of CoCl₂·6H₂O, 0.016% (w/v) of CuSO₄·5H₂O, and 0.012% (w/v) of NiCl₂·6H₂O in 0.1 N hydrochloric acid), followed by 24 hours of shake culture. This culture was passaged 3 times at 24-hour intervals and, after 24 hours of cultivation of the 4th generation, the plasmid retention rate was measured in the following manner.

First, the culture fluid was 10⁸-fold diluted with sterile water, the dilution was distributed, in 10- to 100-µl portions, onto plates (nutrient agar medium (product of DIFCO Laboratories)) without addition of kanamycin, the colonies obtained (100 colonies selected at random) were further replicated onto selective plates (nutrient agar medium (product of DIFCO Laboratories)) supplemented with kanamycin (100 mg/L), and the colonies that had grown were counted. In view of the fact that only the bacteria retaining the plasmid can form colonies on the plates, the number of drug-resistance colonies was taken as an indicator of stability. The results thus obtained are shown in Table 1.

### (Comparative Example 2) Plasmid retention rate in transformant obtained by using plasmid vector pJRD215

A plasmid retention rate test was carried out in the same manner as in Example 4 except that the transformant obtained in Comparative Example 1 was used as the transformant in lieu of the transformant obtained in Example 3 using the plasmid vector pCUP2. The results are shown in Table 1.

**Table 1**

| Microorganism | *R. eutropha H16* | *R. eutropha H16* |
|---|---|---|
| Plasmid | pCUP2 | pJRD215 |
| Number of colonies | 98 | 32 |
| Retention rate | 98% | 32% |

### (Example 5) Construction of plasmid vector for PHA synthase transfer

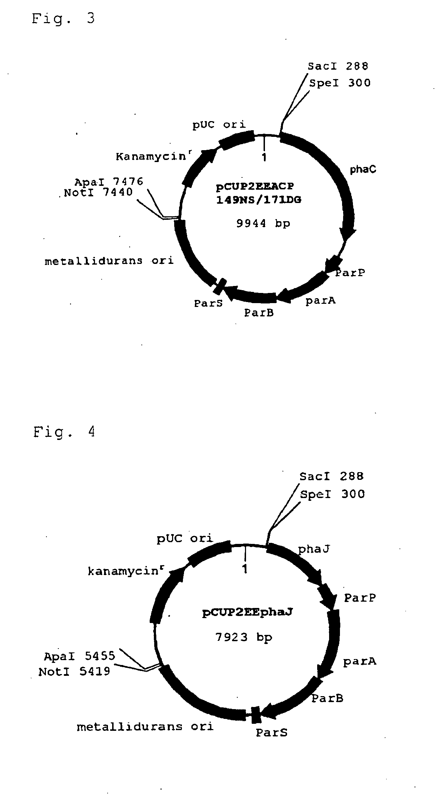

An expression plasmid vector (pCUP2EEACP149NS/171DG) was constructed by inserting, into the plasmid vector (pCUP2) obtained in Example 2, the N149S/D171G mutant, which is an *Aeromonas caviae*-derived PHA synthase mutant gene identified under SEQ ID NO:13, as prepared by *Eco*RI treatment, at the restriction enzyme *Mun*I site of pCUP2 (Fig. 3).

The *Aeromonas caviae*-derived PHA synthase mutant gene, namely the N149S/D171G mutant, was constructed in the following manner. First, pBluescript II KS(-) (product of Toyobo Co.) was treated with *Pst*I, followed by blunting using a DNA blunting kit (product of Takara Bio Inc.) and further followed by ligation, to give a plasmid, pBlue-New, defective in the PstI site. At the *Eco*RI site of this plasmid, there was cloned the d13 fragment excised from pJRD215-EE32d13 (Japanese Kokai Publication Hei-05-93049) with the same enzyme (to give pBlue-d13). Then, using the clone E2-50-derived plasmid (Kichise et al., Appl. Environ. Microbiol., 68:2411-2419 (2002)) as a template and the set of the primers shown under SEQ ID NO:9 and SEQ ID NO:10 or the set of the primers shown under SEQ ID NO:11 and SEQ ID NO:12, two fragments were respectively obtained by amplification by the PCR technique. The conditions were: (1) 2 minutes at 94°C; (2) 30 seconds at 94°C, (3) 30 seconds at 55°C, (4) 2 minutes at 72°C, 25 cycles from (2) to (4); and 5 minutes at 72°C. Equimolar amounts of the two amplified fragments were mixed together, and the PCR reaction was again carried out to join the two fragments together. The conditions were: (1) 5 minutes at 96°C; (2) 2 minutes at 95°C, (3) 1 minute at 72°C, 12 cycles from (2) to (3). The polymerase used was Pyrobest DNA Polymerase (product of Takara Bio Inc.). A DNA fragment having the desired size was excised from an agarose electrophoretic gel, treated with *Pst*I and *Xho*I and cloned in pBlue-d13 treated with the same enzymes in a manner of fragment interchange (to give pBlue-N149S/D171G). The base sequence analysis was carried out using a Perkin Elmer Applied Biosystems DNA sequencer, 310 Genetic Analyzer; it was confirmed that the gene in question is a mutant gene coding for serine in lieu of the 149th amino acid asparagines and for glycine in lieu of the 171st amino acid aspartic acid of the PHA synthase.

The pBlue-N149S/D171G prepared in the above manner was treated with the restriction enzyme *Eco*RI and joined to pCUP2 treated with the restriction enzyme *Mun*I using a DNA ligase (Ligation High, product of Toyobo Co.). A PHA synthase gene-containing plasmid vector, pCUP2EEACP149NS/171DG, was thus constructed.

### (Example 6) Construction of plasmid vector for enoyl-CoA hydratase gene transfer

An expression plasmid vector (pCUP2EEphaJ) was constructed by inserting the *Aeromonas caviae*-derived enoyl-CoA hydratase gene shown under SEQ ID NO:17 as prepared by *Eco*RI treatment into the plasmid vector obtained in Example 2 (pCUP2) at the restriction enzyme *Mun*I site of pCUP2 (Fig. 4). This vector was constructed in the following manner.

First, using, as a template, pJRD215-EE32 (Japanese Kokai Publication Hei-05-93049) containing the enoyl-CoA hydratase gene to be used in the practice of the invention, together with the set of the primers shown under SEQ ID NO: 15 and SEQ ID NO: 16, an amplified fragment containing the enoyl-CoA hydratase gene shown under SEQ ID NO:17 was obtained by amplification using the PCR technique. The conditions were: (1) 2 minutes at 94°C; (2) 10 seconds at 98°C, (3) 10 seconds at 60°C, (4) 1 minutes at 68°C, 30 cycles from (2) to (4); and 3 minutes at 68°C. The polymerase used was LA Taq DNA Polymerase (product of Takara Bio Inc.). Then, this amplified fragment was treated with *Bgl*II and *Afl*II and subjected to ligation treatment with pJRD215-EE32d13 treated in the same manner with *Bgl*II and *Afl*II and then treated with alkaline phosphatase for DNA dephosphorylation treatment; thus, cloning was effected in a manner of replacement of the DNA fragment between the *Bgl*II and *Afl*II sites of pJRD215-EE32d13 (to give pJRD215-EEphaJ). Ligation High (product of Toyobo Co.) was used for the ligation. A DNA fragment containing the enoyl-CoA hydratase gene was prepared from the thus-constructed pJRD215-EEphaJ by treatment with *Eco*RI and ligated with pCUP2 treated with *Mun*I to give pCUP2EEphaJ (Fig. 4). Ligation High (product of Toyobo Co.) was used for the ligation.

### (Example 7) Transformant production using plasmid vector for PHA synthase transfer.

A transformant retaining a plasmid vector for PHA synthase transfer was produced in the same manner as in Example 3 except that a solution prepared from the plasmid vector for PHA synthase transfer (pCUP2EEACP149NS/171DG) obtained in Example 5 was used in lieu of the plasmid pCUP2 solution. The *Ralstonia eutropha* PHB4 strain (Tsuge, T. et al., Macromol. Biosci., Oct. 20; 4(10):963-70 (2004)) incapable of PHA synthesis was used for the transformation.

### (Example 8) Transformant production using plasmid vector for enoyl-CoA hydratase transfer

A transformant retaining a plasmid vector for enoyl-CoA hydratase transfer was produced in the same manner as in Example 3 except that a solution prepared from the plasmid vector for enoyl-CoA hydratase transfer (pCUP2EEphaJ) obtained in Example 6 was used in lieu of the plasmid pCUP2 solution. The *Ralstonia eutropha* PHB4 strain incapable of PHA synthesis was used for the transformation.

### (Example 9) Retention rate of plasmid vector pCUP2EEACP149NS/171DG in transformant

Plasmid retention rate testing was carried out in the same manner as in Example 4 except that the transformant strain obtained in Example 7 was used as the transformant in lieu of the transformant strain obtained in Example 3 by using the plasmid vector pCUP2. The results are shown in Table 2.

**Table 2**

| Microorganism | *R. eutropha PHB -* 4 | *R. eutropha PHB - 4* | *R. eutropha PHB - 4* |
|---|---|---|---|
| Plasmid | pCUP2EEACP149NS/171DG | pCUP2EEphaJ | pJRD215 |
| Number of colonies | 95 | 96 | 30 |
| Retention rate | 95% | 96% | 30% |

### (Example 10) Retention rate of plasmid vector pCUP2EEphaJ in transformant

Plasmid retention rate testing was carried out in the same manner as in Example 4 except that the transformant strain obtained in Example 8 was used as the transformant in lieu of the transformant strain obtained in Example 3 by using the plasmid vector pCUP2. The results are shown in Table 2.

### (Comparative Example 3) Transformant production using plasmid vector pJRD215 and the Ralstonia eutropha PHB4 strain as host

A transformant retaining a plasmid vector pJRD215 was produced in the same manner as in Example 3 except that the pJRD215 containing neither the sequence shown under SEQ ID NO: 18 nor the par region as the plasmid, and using the *Ralstonia eutropha* PHB4 strain as the host.

### (Comparative Example 4) Retention rate of plasmid vector pJRD215, retained in the Ralstonia eutropha PHB4 strain as host, in transformant

Plasmid retention rate testing was carried out in the same manner as in Example 4 except that the transformant strain obtained in Example 3 was used as the transformant in lieu of the transformant strain obtained in Example 3 by using the plasmid vector pCUP2. The results are shown in Table 2.

### (Example 11) Polyester production in transformant strain obtained in Example 7

A Nile red-containing medium (9 g of disodium hydrogen phosphate dodecahydrate, 1.5 g of potassium dihydrogen phosphate, 0.05 g of ammonium chloride, 0.02 g of magnesium sulfate heptahedrate, 0.5 g of fructose, 0.25 ppm of cobalt chloride hexahydrate, 16 ppm of iron (III) chloride hexahydrate, 10.3 ppm of calcium chloride dihydrate, 0.12 ppm of nickel chloride hexahydrate, 0.16 ppm of copper sulfate pentahydrate, 0.5 mg of Nile red, 15 g/L of agar) was seeded with cells of the transformant obtained in Example 7, and cultivation was carried out at 30°C for 2 days. As a result, the colonies turned red; thus, the polyester accumulation in bacterial cells could be confirmed.

### INDUSTRIAL APPLICABILITY

The plasmid vector of the transformant of the invention can be used in bacteria of the species *Cupriavidus necator* as hosts. As it contains neither the mob gene group nor the oriT sequence, in particular, it has no transferability by conjugation and, further, as it contains the par region introduced therein, it can be stably retained in bacteria without any antibiotic-due selective pressure. Transformants obtained by using that vector can stably produce a PHA.

### BRIEF DESCRIPTION OF THE DRAWINGS

Figure 1 shows the genes in pCUP and a restriction enzyme map thereof.
Figure 2 shows the genes in pCUP2 and a restriction enzyme map thereof.
Figure 3 shows the genes in pCUP2EEACP149NS/171DG and a restriction enzyme map thereof.
Figure 4 shows the genes in pCUP2EEphaJ and a restriction enzyme map thereof.

## Claims

1. A transformant which is obtainable by gene transfer into a host bacterium by a recombinant vector,
wherein the host bacterium belongs to the species Cupriavidus necator,
wherein the recombinant vector contains the sequence identified under SEQ ID NO:18 as a part of an origin of replication functioning in bacteria of the genus Cupriavidus as host, and which has neither a mob gene nor an oriT sequence, and
wherein the recombinant vector is obtainable from introduction of, and contains, a vector stabilizing region which is a par region functioning as the par system, and
wherein the recombinant vector contains, as introduced therein, at least one gene involved in the polyhydroxyalkanoate synthesis as selected from the group of genes consisting of the genes for thiolase, the genes for reductase, the genes for polyhydroxybutyrate synthase, the genes for polyhydroxyalkanoate synthase, the genes for acyl-CoA transferase, the genes for enoyl-CoA hydratase, and the genes for acyl-CoA dehydrogenase.

2. The transformant according to claim 1,
wherein the recombinant vector contains the DNA fragment having the sequence identified under SEQ ID NO:19 as introduced therein as the vector stabilizing region.

3. A method for producing a polyhydroxyalkanoate,
which comprises cultivating the transformant according to any one of claims 1 or 2, and recovering and purifying the polyhydroxyalkanoate from the culture.

4. The method for producing a polyhydroxyalkanoate according to claim 3,
wherein the polyhydroxyalkanoate is a copolyester constituted of 3-hydroxybutyric acid and 3-hydroxyhexanoic acid monomer units.

5. The transformant according to claim 1, wherein the host bacterium is either Cupriavidus necator H16 strain (accession number ATCC17699) or Cupriavidus necator PHB-4 strain (accession number DSM541).

6. The transformant according to claim 1 which is used for producing a polyhydroxyalkanoate.

## Patentansprüche

1. Transformante, die mittels Gentransfer in ein Wirtsbakterium durch einen rekombinanten Vektor herstellbar ist,
wobei das Wirtsbakterium zur Spezies Cupriavidus necator gehört,
wobei der rekombinante Vektor die Sequenz gemäß SEQ ID NO: 18 als Teil eines Replikationsursprungs, welcher in Wirtsbakterien des Genus Cupriavidus funktioniert, enthält und weder ein mob Gen noch eine oriT Sequenz besitzt, und
wobei der rekombinante Vektor durch das Hinzufügen einer Vektor-Stabilisierungsregion, welche eine "par"-Region ist, die als "par"-System funktioniert, herstellbar ist und diese enthält, und
wobei der rekombinante Vektor mindestens ein Gen, wie darin eingefügt, enthält, welches bei der Polyhydroxyalkanoat-Synthese involviert ist und ausgewählt ist aus der Gruppe von Genen bestehend aus den Genen für Thiolase, den Genen für Reduktase, den Genen für Polyhydroxybutyrat-Synthase, den Genen für Polyhydroxyalkanoat-Synthase, den Genen für Acyl-CoA-Transferase, den Genen für Enoyl-CoA-Hydratase und den Genen für Acyl-CoA-Dehydrogenase.

2. Transformante gemäß Anspruch 1, wobei der rekombinante Vektor das DNA-Fragment mit der Sequenz gemäß SEQ ID NO: 19, wie darin eingefügt als die Vektor-Stabilisierungsregion, enthält.

3. Verfahren zur Herstellung von Polyhydroxyalkanoat, welches das Kultivieren der Transformante gemäß einem der Ansprüche 1 oder 2 und das Gewinnen und Aufreinigen des Polyhydroxyalkanoats aus der Kultur umfasst.

4. Verfahren zur Herstellung von Polyhydroxyalkanoat gemäß Anspruch 3,
wobei das Polyhydroxyalkanoat ein Copolyester, gebildet aus 3-Hydroxybuttersäure- und 3-Hydroxyhexansäure-Monomer-Einheiten, ist.

5. Transformante gemäß Anspruch 1, wobei das Wirtsbakterium entweder der Cupriavidus necator H16 Stamm (Hinterlegungsnummer ATCC17699) oder der Cupriavidus necator PHB-4 Stamm (Hinterlegungsnummer DSM541) ist.

6. Transformante gemäß Anspruch 1, welche zur Herstellung von Polyhydroxyalkanoat verwendet wird.

## Revendications

1. Transformant qui peut être obtenu par transfert de gène dans une bactérie hôte par un vecteur recombinant,
dans lequel la bactérie hôte appartient à l'espèce Cupriavidus necator,
dans lequel le vecteur recombinant contient la séquence identifiée sous SEQ ID n° 18 en tant qu'une partie d'une origine de réplication fonctionnant dans des bactéries du genre Cupriavidus en tant qu'hôte, et qui n'a ni gène mob, ni séquence oriT, et
dans lequel le vecteur recombinant peut être obtenu à partir d'introduction de, et contient, une région de stabilisation de vecteur qui est une région par fonctionnant en tant que système par, et
dans lequel le vecteur recombinant contient, tel qu'introduit en son sein, au moins un gène impliqué dans la synthèse de polyhydroxyalkanoate tel que sélectionné à partir du groupe de gènes consistant en les gènes pour de la thiolase, les gènes pour de la réductase, les gènes pour de la polyhydroxybutyrate synthase, les gènes pour de la polyhydroxyalkanoate synthase, les gènes pour de l'acyle-CoA transférase, les gènes pour de l'énoyl-CoA hydratase, et les gènes pour de l'acyle-CoA déshydrogénase.

2. Transformant selon la revendication 1,
dans lequel le vecteur recombinant contient le fragment d'ADN ayant la séquence identifiée sous SEQ ID n° 19 tel qu'introduit en son sein en tant que région de stabilisation de vecteur.

3. Procédé pour produire un polyhydroxyalkanoate,
qui comprend la culture du transformant selon l'une quelconque des revendications 1 ou 2, et la récupération et la purification du polyhydroxyalkanoate à partir de la culture.

4. Procédé pour produire un polyhydroxyalkanoate selon la revendication 3,
dans lequel le polyhydroxyalkanoate est un copolyester constitué d'unités monomères d'acide 3-hydroxybutyrique et d'acide 3-hydroxyhexanoïque.

5. Transformant selon la revendication 1, dans lequel la bactérie hôte est soit la souche Cupriavidus necator H16 (numéro d'accès ATCC17699) ou la souche Cupriavidus necator PHB-4 (numéro d'accès DSM541).

6. Transformant selon la revendication 1 qui est utilisé pour produire un polyhydroxyalkanoate.
